(19) Europäisches Patentamt
European Patent Office
Office européen des brevets

(11) Veröffentlichungsnummer: **0 000 784**
**B1**

(12) # EUROPÄISCHE PATENTSCHRIFT

(45) Veröffentlichungstag der Patentschrift: **26.08.81**

(21) Anmeldenummer: **78100633.3**

(22) Anmeldetag: **09.08.78**

(51) Int. Cl.³: **C 07 D 235/26**, A 61 K 31/415, C 07 D 235/02

(54) **4-Hydroxy-2-Benzimidazolinon-derivate, Verfahren zu deren Herstellung sowie diese Verbindungen enthaltende Arzneimittel.**

(30) Priorität: **12.08.77 DE 2736295**
**18.01.78 DE 2801953**

(43) Veröffentlichungstag der Anmeldung:
**21.02.79 Patentblatt 79/4**

(45) Bekanntmachung des Hinweises auf die Patenterteilung:
**26.08.81 Patentblatt 81/34**

(84) Benannte Vertragsstaaten:
**BE CH DE FR GB LU NL SE**

(56) Entgegenhaltungen:
DE - A - 1 493 853
DE - A - 2 615 406
DE - A - 2 700 193

(73) Patentinhaber: **BOEHRINGER MANNHEIM GMBH**
**Sandhofer Strasse 112-132 Postfach 31 01 20**
**D-6800 Mannheim 31-Waldhof (DE)**

(72) Erfinder: **Ross, Carl Heinz, Dr.**
**Rathausstrasse 45**
**D-6806 Viernheim (DE)**
Erfinder: **Friebe, Walter-Gunar, Dr.**
**Oberstrasse 13**
**D-6100 Darmstadt (DE)**
Erfinder: **Kampe, Wolfgang, Dr.**
**Zedernstrasse 49**
**D-6805 Heddesheim (DE)**
Erfinder: **Bartsch, Wolfgang, Dr.**
**Franconviller-Strasse 5**
**D-6806 Viernheim (DE)**
Erfinder: **Roesch, Egon, Dr.**
**Am Oberen Luisenpark 22**
**D-6800 Mannheim 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 000 784 B1

# 4-Hydroxy-2-benzimidazolinon-derivate, verfahren zu deren herstellung sowie diese verbindungen enthaltende arzneimittel

Die vorliegende Erfindung betrifft neue 4 - Hydroxy - 2 - benzimidazolinon - Derivate, Verfahren zu ihrer Herstellung sowie diese Verbindungen enthaltende Arzneimittel.

Die neuen Verbindungen sowie ihre pharmakologisch unbedenklichen Salze bewirken eine Hemmung adrenergischer $\beta$-Rezeptoren und eignen sich daher zur Behandlung oder Prophylaxe bei Herz- und Kreislauferkrankungen.

In der DE—OS 2700193 sind 4 - Hydroxy - 2 - benzimidazolinon - Derivate mit $\beta$ - Rezeptoren - blockierender Wirkung beschrieben, deren anellierter Benzolring unsubstituiert ist.

Die vorliegende Erfindung betrifft basisch substituierte Derivate des 4 - Hydroxy - 2 - benzimidazolinons der allgemeinen Formel I,

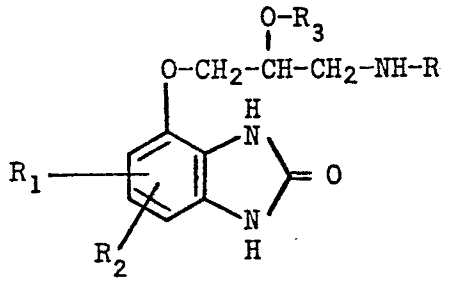

(I),

in der

R einen niederen Alkylrest,

$R_1$ und $R_2$, die gleich oder verschieden sind, jeweils einen niederen, geradkettigen oder verzweigten Alkylrest oder $R_1$ und $R_2$ gemeinsam einen Alkylenrest oder einer der Reste $R_1$ und $R_2$ auch Wasserstoff sein kann und

$R_3$ ein Wasserstoffatom oder einen Acylrest bedeuten, deren pharmakologisch verträgliche Salze, Verfahren zur Herstellung derselben sowie pharmazeutische Zubereitungen, die diese Substanzen enthalten.

Die Verbindungen der allgemeinen Formel I enthalten in der Aminopropoxy-Seitenkette ein optisch aktives Kohlenstoffatom und können daher sowohl in einer racemischen als auch in zwei optisch aktiven Formen auftreten. Gegenstand der vorliegenden Anmeldung sind sowohl die racemischen Formen als auch die optischen Isomeren.

Die niederen Alkylgruppen, die in den Definitionen der Substituenten R, $R_1$ und $R_2$ auftreten, können 1 bis 6, vorzugsweise 1 bis 4 Kohlenstoffatome enthalten. Bevorzugt sind die Methyl-, Isopropyl- und tert-Butylgruppe.

Der Alkylenrest, der gegebenenfalls durch die Substituenten $R_1$ und $R_2$ gebildet werden kann, enthält 2 bis 4 Kohlenstoffatome.

Die Acylgruppen $R_3$ können Säurereste geradkettiger oder verzweigter aliphatischer Carbonsäuren mit 2 bis 6 Kohlenstoffatomen oder aromatischer, gegebenenfalls durch Halogenatome, niedere Alkyl- oder niedere Alkoxygruppen substituierter Carbonsäuren sein. Bevorzugt sind der Acetyl-, Pivaloyl- und Benzoylrest.

Die neuen Verbindungen, deren anellierter Benzolring ein-oder mehrfach durch niedere Alkylgruppen bzw. eine Alkylenbrücke substituiert ist, weisen im Vergleich zum Stand der Technik und zu Handelsprodukten ähnlicher Konstitution und Wirkung eine bessere $\beta$ - Rezeptoren - blockierende Wertung auf.

Zum Beweis wurde beispielsweise im pharmakologischen Test als Vertreter der erfindungsgemäßen Verbindungen die Verbindung des Beispiels 2, das 4 - [2 - Hydroxy - 3 - (2 - propylamino) - propoxy] - 6 - methyl - 2 - benzimidazolinon mit dem 4 - (3 - tert - Butylamino - 2 - hydroxy - propyloxy) - benzimidazol - 2 - on aus der DE—OS 27 00 193 und dem Handelsprodukt Propranolol=1 - Isopropylamino - 3 - (1 - naphthoxy) - 2 - propanol verglichen.

Als Maß für die $\beta$ - Rezeptoren - Blockade wurde an wachen Kaninchen die $DE_{250}$ bestimmt, d. h. die Dosis des $\beta$-Blockers, bei der die Herzfrequenz nach vorheriger Injektion von Isoprenalin nur noch auf 250 Schläge/Min. anstieg.

Die Auswertung der Versuche ergab, daß bei der Anwendung des Handelspräparats eine etwa 100-fache Dosis und bei der Anwendung der bekannten Verbindung etwa die doppelte Dosis wie die der gewählten erfindungsgemäßen Verbindung einzusetzen ist, um die mit Isoprenalin induzierte Herzfrequenzsteigerung unter gleichen Versuchsbedingungen auf 250 Schläge/Min. zu bewirken.

Andere erfindungsgemäße Verbindungen zeigen gleiche oder zum Teil noch besser Versuchsergebnisse.

Die Herstellung der neuen Verbindungen der allgemeinen Formel I ist dadurch gekennzeichnet, daß man in an sich bekannter Wiese entweder.

a) eine Verbindung der allgemeinen Formel II

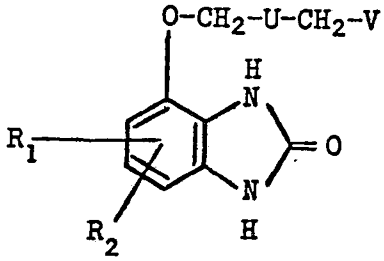

(II),

mit einer Verbindung der allgemeinen Formel III

W—R (III),

umsetzt,

in denen R, $R_1$ und $R_2$ die oben angegebene Bedeutung haben, während U für die Gruppe

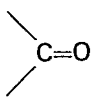 oder 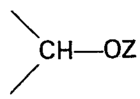

steht, wobei Z die Bedeutung des Substituenten $R_3$ besitzt oder auch zusammen mit V eine Einfachbindung sein kann und einer der Reste V und W eine Aminogruppe und der andere einen reaktiven Rest darstellen,

und

falls U die Gruppe 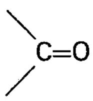 bedeutet, anschließend reduziert

oder

b) eine Verbindung der allgemeinen Formel IV

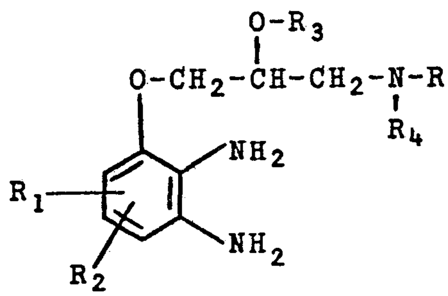

(IV),

mit einer Verbindung der allgemeinen Formel V

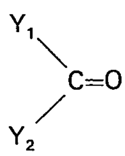

(V)

umsetzt,

in denen R, $R_1$, $R_2$ und $R_3$ die oben angegebene Bedeutung haben, während $R_4$ ein Wasserstoffatomen oder eine abspaltbare Schutzgruppe darstellt, $Y_1$ und $Y_2$ gleich oder Verschieden sind und jeweils einen reaktiven Rest beschreiben,

und anschließend eine gegebenenfalls vorhandene Schutzgruppe $R_4$ wieder abspaltet,

oder

c) eine Verbindung der allgemeinen Formel VI

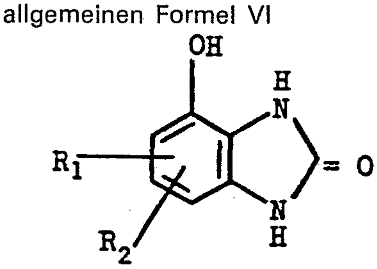

(VI),

mit einer Verbindung der allgemeinen Formel VII

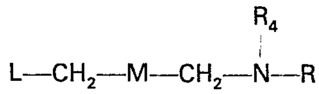

(VII),

umsetzt,

in denen R, $R_1$, $R_2$ und $R_4$ die oben angegebene Bedeutung haben, während M für die Gruppen

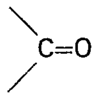 oder 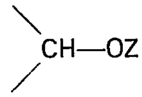

steht, wobei Z die Bedeutung des Substituenten $R_3$ besitzt oder auch zusammen mit L eine Einfachbindung sein kann, und der Rest L einen reaktiven Rest darstellt,

falls M die Gruppe

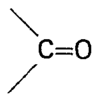

bedeutet, anschließend reduziert, und eine gegebenenfalls vorhandene Schutzgruppe $R_4$ wieder abspaltet

und anschließend an die Umsetzungen gegebenenfalls Verbindungen der allgemeinen Formel I in pharmakologisch verträgliche Salze überführt, wobei man gegebenenfalls vorher in Verbindungen der allgemeinen Formel I, in denen $R_3$ Wasserstoff bedeutet, die Hydroxygruppe acyliert.

$Y_1$ und $Y_2$ in Verbindungen der allgemeinen Formel V stehen für alle Reste, die mit den beiden primären Aminogruppen in Verbindungen der allgemeinen Formel IV sum Imidazolinring reagieren können. Solche Reste sind vorzugsweise Halogenatome, wie Brom oder Chlor, Amino-, Imidazolyl-, Niederalkoxy-, niedere Acyloxy- und Phenoxygruppen.

Beispielsweise können als Verbindungen der allgemeinen Formel V Carbonylhalogenide, Harnstoff, N,N'-Carbonyldiimidazol eingesetzt werden.

Die erfindungsgemäßen Verfahren werden zweckmäßig in einem unter den Reaktionsbedingungen inerten Lösungsmittel, z.B. Wasser, Ethanol, Dioxan oder Dimethylformamid, gegebenenfalls in Gegenwart eines säurebindenden Mittels, durchgeführt. Die Umsetzungen können auch nach Mischen der Reaktionskomponenten ohne Lösungsmittel erreicht werden. Die Reaktionen werden durch Stehenlassen bei Raumtemperatur oder durch Erwärmen, gegebenenfalls unter einer Schutzgasatmosphäre ausgeführt.

Die gegebenenfalls durchzuführende Reduktion der Gruppe

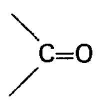

erfolgt durch katalytische Hydrierung mit Edelmetall- oder Nickelkatalysatoren oder mittels komplexer Metallhydride, wie z.B. Natriumborhydrid.

Als leicht abspaltbare Schutzgruppen können im Prinzip alle in der peptidchemie zum intermediären Schutz von Aminogruppen verwendeten Schutzgruppen eingesetzt werden, die nach erfolgter umsetzung wieder entfernt werden können. Mit großem Vorteil werden bei der vorliegenden Umsetzung gemäß Verfahren b) und c) die Benzyl- und Carbobenzoxygruppe eingeführt, die sich nach der Reaktion der Verbindungen der allgemeinen Formel IV bzw. VI mit Substanzen der allgemeinen Formel V bzw. VII ohne weiteres in an sich bekannter Weise hydrogenolytisch abspalten lassen.

Die Verbindungen der allgemeinen Formel IV können beispielsweise hergestellt werden, indem man Verbindungen der allgemeinen Formel VIII

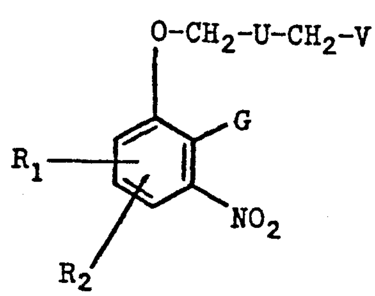

(VIII),

in der $R_1$, $R_2$, U und V die oben angegebene Bedeutung haben, während G für die Amino- oder Nitrogruppe steht,
mit Verbindungen der allgemeinen Formel III analog Verfahren a) umsetzt und die so erhaltenen Substanzen reduziert. Die Reduktion erfolgt in an sich bekannter Weise, vorzugsweise durch katalytische Hydrierung. Die hierbei entstehenden rohen o-Phenylendiaminderivate der allgemeinen Formel IV werden vorteilhaft ohne weitere Reinigung als mineralsaure Salze als Ausgangsprodukte beim Verfahren b) eingesetzt.

Die Verbindungen der allgemeinen Formel VIII sind entweder beschriebene Substanzen oder können leicht aus beschriebenen Substanzen nach bekannten Methoden hergestellt werden.

Die gegebenenfalls durchzuführende nachträgliche Acylierung von Verbindungen der allgemeinen Formel I, in der $R_3$ ein Wasserstoffatom bedeutet, kann in üblicher Weise durch Umsetzung mit einem reaktiven Säurederivat, z.B. Säurehalogenid, Säureazid oder Säureanhydrid, gegebenenfalls in Gegenwart eines säurebindenden Mittels, z.B. Pyridin, in einem Lösungsmittel, z.B. Aceton, Benzol, Dimethylformamid, oder auch in überschüssiger Säure erfolgen.

Die erfindungsgemäßen Verbindungen der Formel I können in Form eines racemischen Gemisches anfallen. Die Trennung des Racemats in die optisch aktiven Formen geschieht nach an sich bekannten Methoden über die diastereomeren Salze aktiver Säuren, wie z.B. Weinsäure, Apfelsäure oder Camphersulfonsäure.

Die neuen verbindungen der allgemeinen Formel I fallen unter den Reaktionsbedingungen der beschriebenen Verfahren vorwiegend als Säureadditionssalze an, z.B. als Hydrochloride, und können nach beschriebenen Methoden ohne weiteres in die freien Basen überführt werden.

Zur Überführung der Verbindungen der allgemeinen Formel I in ihre pharmakologisch unbedenklichen Salze setzt man diese, vorgugsweise in einem organischen Lösungsmittel, mit der äquivalenten Menge einer anorganischen oder organischen Säure, z.B. Salzsäure, Bromwasserstoffsäure, Phosphorsäure, Schwefelsäure, Essigsäure, Citronensäure, Maleinsäure, um.

Zur Herstellung von Arzneimitteln werden die Substanzen I in an sich bekannter Weise mit geeigneten pharmazeutischen Trägersubstanzen, Aroma-, Geschmacks- und Farbstoffen gemischt und beispielsweise als Tabletten oder Dragees ausgeformt oder unter Zugabe entsprechender Hilfsstoffe in Wasser oder Öl, wie z.B. Olivenöl, suspendiert oder gelöst.

Die erfindungsgemäßen neuen Substanzen I und ihre Salze können in flüssiger oder fester Form enteral oder partenteral appliziert werden. Als Injektionsmedium kommt vorzugsweise Wasser zur Anwendung, welches die bei Injektionslösungen üblichen Zusätze wie Stabilisierungsmittel, Lösungsvermittler oder Puffer enthält. Derartige Zusätze sind z.B. Tartrat- und Citratpuffer, Ethanol, Komplexbildner (wie Ethylendiamintetraessigsäure und deren nicht-toxische Salze), hochmolekulare Polymere (wie flüssiges Polyethylenoxid) zur Viskositätsregulierung. Feste Trägerstoffe sind z.B. Stärke, Lactose, Mannit, Methylcellulose, Talkum, hochdisperse Kieselsäuren, höhermolekulare Fettsäuren (wie Stearinsäure), Gelatine, Agar-Agar, Calciumphosphat, Magnesiumstearat, tierische und pflanzliche Fette und feste hochmolekulare Polymere (wie Polyethylenglykole). Für orale Applikation geeignete Zubereitungen können gewünschtenfalls Geschmacks- und Süßstoffe enthalten.

Bevorzugt im Sinne der vorliegenden Anmeldung sind außer den in den Beispielen genannten Verbindungen die folgenden:

7 - tert - Butyl - 4 - (2 - hydroxy - 3 - tert - butylamino - propoxy) - 2 - benzimidazolinon,
4 - (2 - Pivaloyloxy - 3 - tert - butylamino - propoxy) - 6 - methyl - 2 - benzimidazolinon.

Die Erfindung wird durchdie folgenden Beispiele näher erläutert. Sie zeigen einige der zahlreichen möglichen Verfahrensvarianten, die zur Synthese der erfindungsgemäßen Verbindungen verwendet werden können. Sie stellen jedoch keine Einschränkung des Erfindungsgegenstandes dar.

## Beispiel 1

*4 - (2 - Hydroxy - 3 - tert - butylamino - propoxy) - 6 - methyl - 2 - benzimidazolinon*

In eine Lösung von 5,8 g 2,3 - Diamino - 1 - (2 - hydroxy - 3 - tert - butylaminopropoxy) - 5 - methyl - benzol - trihydrochlorid in 150 ml Wasser wird ca. 30 Minuten in mäßigem Strom Phosgen eingeleitet. Nach Spülen mit Stickstoff engt man zur Trockene ein und kristallisiert aus Ethanol 3,04 g (61% d. Th.) Titelverbindung vom Fp. 280—281°C (als Hydrochlorid).

Die als Zwischenprodukt benötigte Diaminoverbindung kann auf folgendem Weg dargestellt werden:

Durch Nitrierung von vorher acetyliertem 2 - Amino - 5 - methylphenol (Fp. 156—158°C) bei 30°C mit Acetanhydrid in Eisessig erhält man 1 - Acetoxy - 2 - acetamido - 3 - nitro - 5 - methylbenzol mit Fp. 163—165°C.

Verseifen mit 2 N Salzsäure liefert 2 - Amino - 5 - methyl - 3 - nitrophenol vom Fp. 197—199°C.

Aus der vorstehenden Verbindung erhält man nach Umsetzen mit 3 - Chlor - 1,2 - epoxypropan und 25 proz. Natronlauge bei 75°C das 2 - (2,3 - Epoxy - propoxy) - 4 - methyl - 6 - nitro - anilin mit Fp. 90 bis 91°C.

Reaktion der vorstehenden Verbindung mit tert-Butylamin in Ethanol und anschließende katalytische Hydrierung über Platindioxid führt nach Ansäuern mit Salzsäure zur amorphem 2,3 - Diamino - 1 - (2 - hydroxy - 3 - tert - butylamino - propoxy) - 5 - methylbenzol - trihydrochlorid.

Beispiel 2

*4 - [2 - Hydroxy - 3 - (2 - propylamino) - propoxy] - 6 - methyl - 2 - benzimidazolinon*

Analog dem in Beispiel 1 genannten Verfahren erhält man aus 2,3 - Diamino - 1 - [2 - hydroxy - 3 - (2 - propylamino) - propoxy] - 5 - methylbenzoltrihydrochlorid die Titelverbindung mit Fp. 286—288°C (als Hydrochlorid).

Das als Zwischenprodukt eingesetzte Diamin wird durch Umsetzung des in Beispiel 1 beschriebenen 2 - (2,3 - Epoxy - propoxy) - 4 - methyl - 6 - nitroanilins mit 2- Propylamin zu 2 - [2 - Hydroxy - 3 - (2 - propylamino) - propoxy] - 4 - methyl - 6 - nitroanilin und anschließende katalytische Hydrierung erhalten.

Beispiel 3

*4 - (2 - Hydroxy - 3 - tert - butylamino - propoxy) - 7 - methyl - 2 - benzimidazolinon*

8,0 g 2,3 - Diamino - 1 - (2 - hydroxy - 3 - tert - butylamino - propoxy) - 4 - methylbenzol - trihydrochlorid löst man in 220 ml Wasser. Man leitet Phosgen ein und saugt die ausgefallenen Kristalle ab. Nach Umkristallisation aus Ethanol unter Zusatz von Aktivkohle erhält man 3,55 g (54% d. Th.) Titelverbindung mit Zersetzungspunkt 312°C (als Hydrochlorid).

Die in obiger Reaktion eingesetzte Zwischenstufe kann auf folgendem Weg hergestellt werden:

2,3 - Dinitro - 4 - methyl - phenol (H. E. Dadswell und J. Kenner, J. Chem. Soc. *1927*, 583) wird mit 3 - Chlor - 1,2 - epoxypropan und 25 proz. Natronlauge umgesetzt. Man erhält in 78% Ausbeute 2,3 - Dinitro - 1 - (2,3 - epoxy - propoxy) - 4 - methyl - benzol vom Fp. 121—123°C.

Die vorstehende Verbindung wird in siedendem Ethanol mit tert-Butylamin zu 2,3 - Dinitro - 1 - (2 - hydroxy - 3 - tert - butylamino - propoxy) - 3 - methyl - benzol mit Fp. 104—106°C umgesetzt.

Durch katalytische Hydrierung über Platindioxid erhält man daraus amorphes 2,3 - Diamino - 1 - (2 - hydroxy - 3 - tert - butyl - amino - propoxy) - 4 - methyl - benzol, das als Trihydrochlorid isoliert wird.

Beispiel 4

*4 - [2 - Hydroxy - 3 - (2 - propylamino) - propoxy] - 7 - methyl - 2 - benzimidazolinon*

Analog dem in Beispiel 3 genannten Verfahren erhält man aus 2,3 - Diamino - 1 - [2 - hydroxy - 3 - (2 - propylamino) - propoxy] - 4 - methyl - benzol - trihydrochlorid die Titelverbindung mit Fp. 305—307°C (als Hydrochlorid).

Das als Zwischenprodukt eingesetzte Diamin wird durch Umsetzung des im Beispiel 3 beschriebenen 2,3 - Dinitro - 1 - (2,3 - epoxy - propoxy) - 4 - methyl - benzols mit 2-Propylamin zu 2,3 - Dinitro - 1 - [2 - hydroxy - 3 - (2 - propylamino) - propoxy] - 4 - methyl - benzol vom Fp. 122—124°C und anschließende katalytische Hydrierung dargestellt.

Beispiel 5

*4 - [2 - Benzoyloxy - 3 - tert - butylamino - propoxy] - 7 - methyl - 2 - benzimidazolinon*

4 - (2 - Hydroxy - 3 - tert - butylamino - propoxy) - 7 - methyl - 2 - benzimidazolinon (dargestellt aus dem Hydrochlorid (Beispiel 3) und methanolischem Natriummethylat) rührt man 5 Tage bei Raumtemperatur mit der äquimolekularen Menge Benzoesäureazid in Dimethylformamid. Die kristalline Abscheidung wird mit etherischer Salzsäure versetzt und nach Einengen aus Ethanol umkristallisiert. Man erhält die Titelverbindung vom Fp. 216—218°C (als Hydrochlorid).

Beispiel 6

*6 - tert - Butyl - 4 - [2 - hydroxy - 3 - (2 - propylamino) - propoxy] - 2 - benzimidazolinon*

In eine Lösung von 5,5 g 5 - tert - Butyl - 2,3 - diamino - 1 - [2 - hydroxy - 3 - (2 - propylamino) - propoxy] - benzol - trihydrochlorid in 50 ml Wasser leitet man bei 20—25°C überschüssiges Phosgen ein, spült gründlich mit Stickstoff nach, engt ein und nimmt den Rückstand in Isopropanol auf. Dabei scheiden sich 2,3 g (43% d. Th.) Titelverbindung, Zersetzungspunkt oberhalb 280°C, nach kurzer Zeit kristallin ab (als Hydrochlorid).

Die als Zwischenprodukt verwendete Diaminoverbindung kann auf folgendem Wag hergestellt werden:

Durch Hydrierung von 3 - tert - Butyl - 6 - nitro - phenol in wässirigethanolischer Lösung über palladiumkohle erhält man 6 - Amino - 3 - tert - butylphenol vom Zersetzungspunkt 206—208°C, das nach Acetylierung mit Acetanhydrid in Essigester/Pyridin 4 - Acetamido - 3 - acetoxy - 1 - tert - butyl - benzol vom 109—110°C liefert.

Die Umsetzung der vorstehenden Verbindung mit Salpetersäure in Acetanhydrid ergibt 2 - Acetamido - 3 - acetoxy - 5 - tert - butylnitrobenzol vom Fp. 200—201°C.

Bei der sauren Verseifung der vorstehenden Verbindung mit verd. Salzsäure isoliert man 2 - Amino - tert - butyl - 3 - nitrophenol vom Fp. 180—181°C.

Aus der vorstehenden Verbindung entsteht bei der Reaktion mit 3 - Chlor - 1,2 - epoxy - propan in 2 N Natronlauge 4 - tert - Butyl - 2 - (2,3 - epoxy - propoxy) - 6 - nitro - anilin als dunkles Öl.

Durch Reaktion der vorstehenden Verbindung mit 2-Propylamin erhält man 4 - tert - Butyl - 6 - nitro - 2 - [2 - hydroxy - 3 - (2 - propylamino)propoxy] - anilin, das als amorphes Hydrochlorid isoliert wird.

Die Hydrierung der vorstehenden Verbindung in ethanolischer Lösung über Platindioxid liefert 5 - tert - Butyl - 2,3 - diamino - 1 - [2 - hydroxy - 3 - (2 - propylamino) - propoxy] - benzol, das als amorphes Trihydrochlorid isoliert wird.

Beispiel 7

*6 - tert - Butyl - 4 - (2 - hydroxy - 3 - tert - butylamino - propoxy)2 - benzimidazolinon*

Analog dem in Beispiel 6 beschriebenen Verfahren erhält man die Titelverbindung als Hydrochlorid mit Zersetzungspunkt oberhalb 300°C aus 5 - tert - Butyl - 2,3 - diamino - 1 - (2 - hydroxy - 3 - tert - butylamino - propoxy) - benzol - trihydrochlorid.

Die als Zwischenprodukt verwendete Diaminoverbindung kann über folgende Stufen synthetisiert werden:

Durch Reaktion von 4 - tert - Butyl - 2 - (2,3 - epoxy - propoxy) - 6 - nitroanilin mit tert-Butylamin erhält man 4 - tert - Butyl - 2 - (2 - hydroxy - 3 - tert - butylamino - propoxy) - 6 - nitro - anilin, das als Hydrochlorid vom Zersetzungspunkt 115—120°C isoliert wird.

Die Hydrierung der vorstehenden Verbindung in ethanolischer Lösung über Platindioxid ergibt 5 - tert - Butyl - 2,3 - diamino - 1 - (2 - hydroxy - 3 - tert - butylamino - propoxy) - benzol, das als amorphes Trihydrochlorid isoliert wird.

Beispiel 8

*6,7 - Dimethyl - 4 - (2 - hydroxy - 3 - tert - butylamino - propoxy) - 2 - benzimidazolinon*

In die wäßrige Lösung von 7,2 g 2,3 - Diamino - 4,5 - dimethyl - 1 - (2 - hydroxy - 3 - tert - butylamino - propoxy) - benzol - trihydrochlorid wird wie in den vorstehenden Beispielen beschrieben Phosgen eingeleitet. Der nach Einengen erhaltene Rückstand wird aus 30 ml Ethanol unter Zusatz von Aktivkohle umkristallisiert. Nach Versetzen mit 20 ml Essigester erhält man 1,1 g (17% d. Th.) Titelverbindung vom Fp. 308—310°C (als Hydrochlorid).

Die als Zwischenstufe benötigte Diaminoverbindung wird auf folgendem Wege aus dem 2 - Amino - 4,5 - dimethyl - phenol [Lit.: E. Diepolder, Chem. Ber. *42*, 2916 (1909)] synthetisiert:

Man acetyliert das vorstehende Phenol mit Acetanhydrid in Essigester/Pyridin zu 2 - Acetamido - 1 - acetoxy - 4,5 - dimethylbenzol (Fp. 156—158°C), das in Acetanhydrid mit 100 proz. Salpetersäure in Acetanhydrid bei 20°C nitriert wird. Aus dem Nitriergemisch isoliert man in 43% Ausbeute das 2 - Acetamido - 1 - acetoxy - 4,5 - dimethyl - 3 - nitro - benzol vom Fp. 209—211°C.

Nach Verseifen der vorstehenden Verbindung in 2 N Salzsäure isoliert man 2 - Amino - 4,5 - dimethyl - 3 - nitro - phenol mit Fp. 176—178°C.

Die vorstehende Verbindung wird mit methanolischem Natrium-methylat in das Natrium-Salz überführt. Dieses wird in Dioxan/Dimethylformamid mit überschüssigem 3 - Chlor - 1,2 - epoxypropan bei 75°C umgesetzt. Nach Einengen wird der Rückstand in Chloroform aufgenommen, mit Wasser und Aktivkohle behandelt und vom Lösungsmittel befreit.

Dieser amorphe Rückstand von 2 - Amino - 4,5 - dimethyl - 1 - (2,3 - epoxy - propoxy) - 3 - nitro - benzol reagiert in siedendem Ethanol mit tert-Butylamin zu 2 - Amino - 4,5 - dimethyl - (2 - hydroxy - 3 - tert - butylamino - propoxy) - 3 - nitro - benzol.

Die vorstehende Verbindung wird quantitativ in Ethanol über Platindioxid zu 2,3 - Diamino - 4,5 - dimethyl - 1 - (2 - hydroxy - 3 - tert - butylamino - propoxy) - benzol hydriert, das als Trihydrochlorid isoliert wird.

Beispiel 9

*6,7 - Dimethyl - 4 - [2 - hydroxy - 3 - (2 - propylamino) - propoxy] - 2 - benzimidazolinon*

Analog dem in Beispiel 8 angegebenen Verfahren synthetisiert man die Titelverbindung als Hydrochlorid vom Fp. 338—340°C aus 2,3 - Diamino - 4,5 - dimethyl - 1 - [2 - hydroxy - 3 - (2 - propylamino) - propoxy] - benzoltrihydrochlorid.

Die als Zwischenstufe benötigte Diaminoverbindung wird durch Umsetzung von 2 - Amino - 4,5 - dimethyl - 1 - (2,3 - epoxy - propoxy) - 3 - nitrobenzol (Beispiel 8) mit 2-Propylamin in siedendem Ethanol und anschließende katalytische Hydrierung erhalten.

Beispiel 10

*4 - (2 - Hydroxy - 3 - tert - butylamino - propoxy) - 6,7 - cyclopenteno - 2 - benzimidazolinon*

Analog dem in Beispiel 6 beschriebenen Verfahren erhält man die Titelverbindung als Hydrochlorid vom Zersetzungspunkt oberhalb 280°C aus 4,5 - Diamino - 6 - (2 - hydroxy - 3 - tert - butylamino - propoxy) - indan - trihydrochlorid.

Die als Zwischenprodukt verwendete Diaminoverbindung kann auf folgendem Weg hergestellt werden:

Die Acetylierung von 6 - Amino - 5 - indanol mit Acetanhydrid in Essigester/Pyridin liefert 6 - Acetamido - 5 - acetoxy - indan vom Fp. 157—158°C.

Die Umsetzung der vorstehenden Verbindung mit Salpetersäure in Acetanhydrid ergibt 5 - Acetamido - 6 - acetoxy - 4 - nitroindan vom Fp. 168—170°C.

Bei der sauren Verseifung der vorstehenden Verbindung mit verd. Salzsäure isoliert man 6 - Amino - 7 - nitro - 5 - indanol vom Fp. 196—198°C.

Aus der vorstehenden Verbindung entsteht bei der Reaktion mit 3 - Chlor - 1,2 - epoxy - propan in verd. Natronlauge 5 - Amino - 6 - (2,3 - epoxy - propoxy) - 4 - nitro - indan als dunkles Öl.

Durch Reaktion der vorstehenden Verbindung mit tert-Butyl-amin erhält man 5 - Amino - 6 - (2 - hydroxy - 3 - tert - butylamino - propoxy) - 4 - nitro - indan, das als amorphes Hydrochlorid isoliert wird.

Die Hydrierung der vorstehenden Verbindung in ethanolischer Lösung über Platindioxid liefert 4,5 - Diamino - 6 - (2 - hydroxy - 3 - tert - butylamino - propoxy) - indan, das als amorphes Trihydrochlorid isoliert wird.

**Patentansprüche**

1. 4 - Hydroxy - 2 - benzimidazolinon - Derivate der allgemeinen Formel I

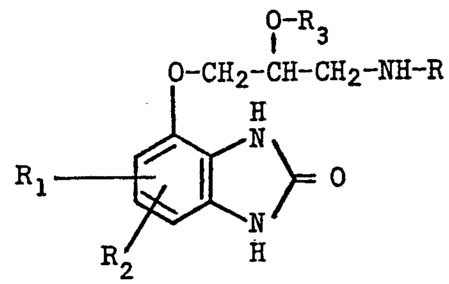

(I)

in der

R einen niederen Alkylrest,

$R_1$ und $R_2$, die gleich oder verschieden sind, jeweils einen niederen, geradkettigen oder verzweigten Alkylrest oder $R_1$ und $R_2$ gemeinsam einen Alkylenrest oder einer der Reste $R_1$ und $R_2$ auch Wasserstoff sein kann und

$R_3$ ein Wasserstoffatom oder einen Acylrest bedeuten, sowie deren pharmakologisch verträgliche Salze.

2. Verfahren zur Herstellung von 4 - Hydroxy - 2 - benzimidazolinon - Derivaten der allgemeinen Formel I

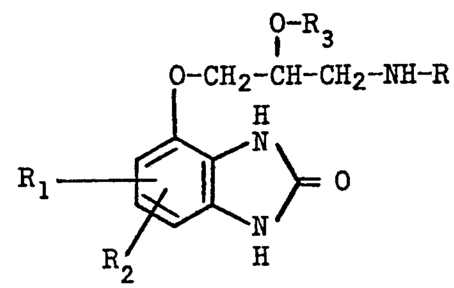

(I),

in der

R einen niederen Alkylrest,

$R_1$ und $R_2$, die gleich oder verschieden sind, jeweils einen niederen, geradkettigen oder verzweigten Alkylrest oder $R_1$ und $R_2$ gemeinsam einen Alkylenrest oder einer der Reste $R_1$ und $R_2$ auch Wasserstoff sein kann und

$R_3$ ein Wasserstoffatom oder einen Acylrest bedeuten, sowie deren pharmakologisch verträglichen Salze, dadurch gekennzeichnet, daß man in an sich bekannter Weise entweder

a) eine Verbindung der allgemeinen Formel II

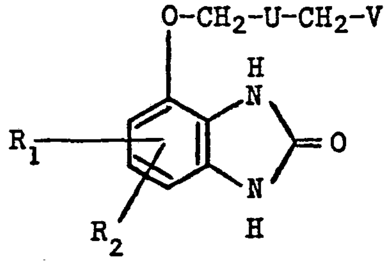

(II),

mit einer Verbindung der allgemeinen Formel III

W—R (III),

umsetzt,

in denen R, $R_1$ und $R_2$ die oben angegebene Bedeutung haben, während U für die Gruppe

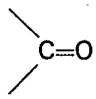 oder 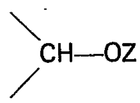

steht, wobei Z die Bedeutung des Substituenten $R_3$ besitzt oder auch zusammen mit V eine Einfachbindung sein kann und einer der Reste V und W eine Aminogruppe und der andere einen reaktiven Rest darstellen,

und

falls U die Gruppe 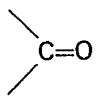 bedeutet, anschließend reduziert

oder

b) eine Verbindung der allgemeinen Formel IV

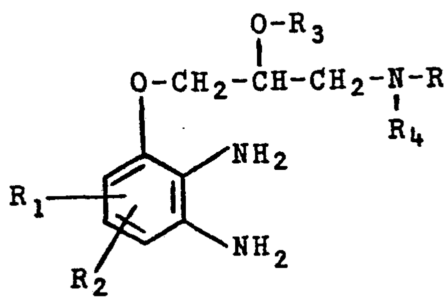

(IV),

mit einer Verbindung der allgemeinen Formel V

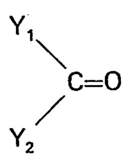

(V)

umsetzt,

in denen R, $R_1$, $R_2$ und $R_3$ die oben angegebene Bedeutung haben, während $R_4$ ein Wasserstoffatom oder eine abspaltbare Schutzgruppe darstellt, $Y_1$ und $Y_2$ gleich oder verschieden sind und jeweils einen reaktiven Rest beschreiben,

und anschließend eine gegebenenfalls vorhandene Schutzgruppe $R_4$ weider abspaltet,

oder

c) eine Verbindung der allgemeinen Formel VI

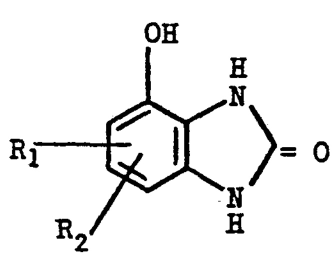

(VI),

mit einer Verbindung der allgemeinen Formel VII

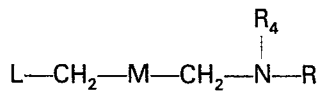

(VII),

umsetzt,

in denen R, $R_1$, $R_2$ und $R_4$ die oben angegebene Bedeutung haben, während M für die Gruppen

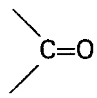 oder 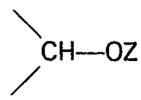

steht, wobei Z die Bedeutung des Substituenten $R_3$ besitzt oder auch zusammen mit L eine Einfachbindung sein kann, und der Rest L einen reaktiven Rest darstellt, falls M die Gruppe

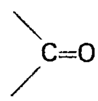

bedeutet, anschließend reduziert, und eine gegebenenfalls vorhandene Schutzgruppe $R_4$ wieder abspaltet und anschließend an die Umsetzungen gegebenenfalls Verbindungen der allgemeinen Formel I in pharmakologisch verträgliche Salze überführt, wobei man gegebenenfalls vorher in Verbindungen der allgemeinen Formel I, in denen $R_3$ Wasserstoff bedeutet, die Hydroxygruppe acyliert.

3. Arzneimittel enthaltend eine Verbindung gemäß Anspruch 1 sowie an sich bekannte pharmakologisch unbedenkliche Träger- und Hilfstoffe.

4. Verbindungen gemäß Anspruch 1 zur Behandlung oder Prophylaxe von Herz- und Kreislauferkrankungen.

**Revendications**

1. Dérivés de la 4 - hydroxy - 2 - benzimidazolinone de formule générale I

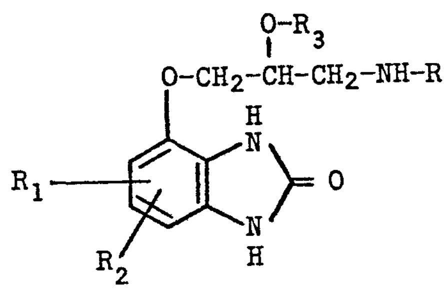

(I)

dans laquelle:

R est un reste alkyle inférieur,

$R_1$ et $R_2$, pouvant être égaux ou différents, sont chacun un reste alkyle inférieur à chaîne droite ou ramifiée, ou $R_1$ et $R_2$ forment ensemble un reste alkylène, ou encore un des deux restes $R_1$ et $R_2$ est de l'hydrogène, et,

$R_3$ est un atome d'hydrogène ou un reste acyle, ainsi que leurs sels pharmacologiquement tolérables.

2. Procédé pour la préparation de dérivés de la 4 - hydroxy - 2 - benzimidazolinone de formule générale I

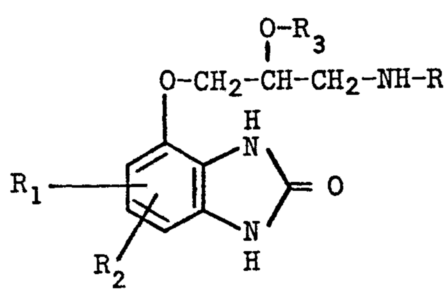

(I)

dans laquelle:

R est un reste alkyle inférieur,

$R_1$ et $R_2$, pouvant être égaux ou différents, sont chacun un reste alkyle inférieur à chaîne droite ou ramifiée, ou

$R_1$ et $R_2$ forment ensemble un reste alkylène ou encore un des deux restes $R_1$ et $R_2$ est d l'hydrogène, et,

$R_3$ est un atome d'hydrogène ou un reste acyle, ainsi que leurs sels pharmacologiquement tolérables, caractérisé en ce que l'on fait réagir de façon en soi connue

a) un composé de formule générale II

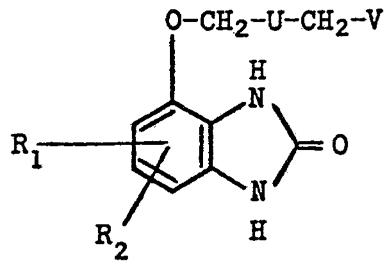

(II)

avec un composé de formule générale III

W—R (III)

dans lesquelles R, $R_1$ et $R_2$ ont la signification ci-dessus donnée, tandis que U désigne le groupe

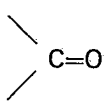 ou 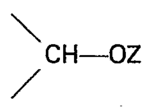

où Z a la signification du substituant $R_3$, ou bien forme ensemble avec V un composé simple, l'un des deux restes V et W représentant un groupe aminogène et l'autre un reste réactif, et, dans le cas où U est le groupe

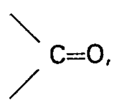

on les soumet à une réduction, ou

b) un composé de formule générale IV

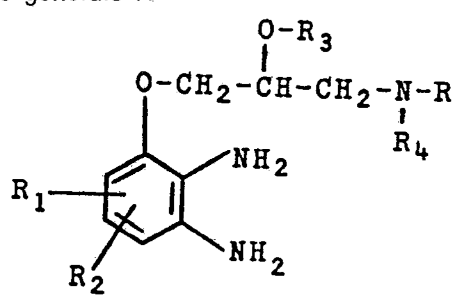

(IV)

avec un composé de formule V

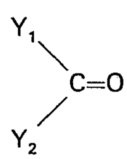

(V)

dans lesquelles:

R, $R_1$, $R_2$ et $R_3$ ont la signification ci-dessus indiquée, tandis que $R_4$ est un atome d'hydrogène ou un groupe de protection éliminable, $Y_1$ et $Y_2$ sont identiques ou différents et chacun un reste réactif; et on élimine ensuite un groupe de protection $R_4$ évuentuellement présent, ou

c) un composé de formule générale VI

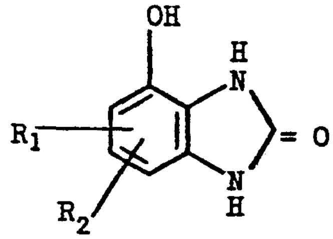

(VI)

avec un composé de formule générale VII

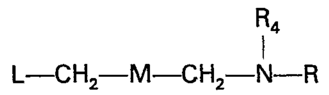

(VII),

dans lesquelles R, $R_1$, $R_2$ et $R_4$ ont la signification ci-dessus indiquée, tandis que M désigne le groupe

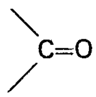 ou 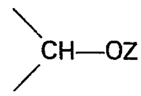

où Z signifie un substituant $R_3$ ou bien forme ensemble avec L un composé simple, et L est un reste réactif, et dans le cas où M est le groupe

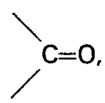

ou effectue ensuite une réduction, et on élimine de nouveau un groupe de protection $R_4$ éventuellement présent,

et en ce qu'après ces réactions on transforme éventuellement les composés de formule générale I en sels pharmacologiquement tolérables, après avoir transformé éventuellement par acylation la fonction hydroxyle dans les composés de formule générale I dans lesquelles $R_3$ est de l'hydrogène.

3. Médicaments contenant un composé selon la revendication 1 ainsi que des substances auxiliaires et de support connues, pharmacologiquement acceptables.

4. Dérivés selon la revendication 1 pour le traitement ou pour le prophylaxie des maladies du coeur et de la circulation.

**Claims**

1. 4 - Hydroxy - 2 - benzimidazolinone derivatives of the general formula I

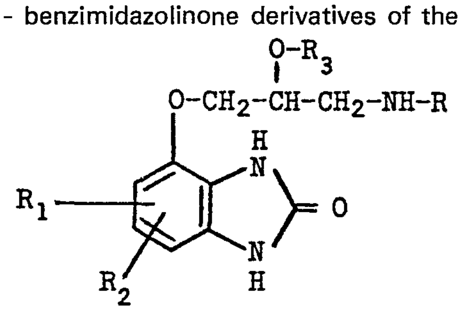

(I),

in which R signifies a lower alkyl radical, $R_1$ and $R_2$, which are the same or different, are each a lower straight-chained or branched alkyl radical or $R_1$ and $R_2$ can together be an alkylene radical and one of the radicals $R_1$ and $R_2$ can also be hydrogen and $R_3$ a hydrogen atom or an aceyl radical; as well as their pharmacologically acceptable salts.

2. Process for the preparation of 4 - hydroxy - 2 - benzimidazolinone derivatives of the general formula I

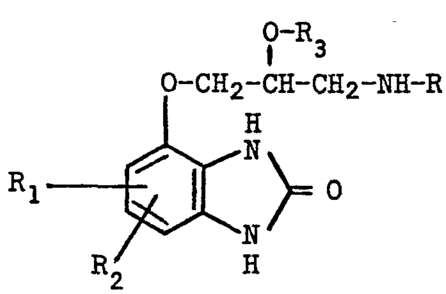

(I),

in which R signifies a lower alkyl radical, $R_1$ and $R_2$, which are the same or different, are each a lower straight-chained or branched alkyl radical or $R_1$ and $R_2$ can together be an alkylene radical and one of the radicals $R_1$ and $R_2$ can also be hydrogen and $R_3$ a hydrogen atom or an acyl radical; as well as of their pharmacologically acceptable salts, characterised in that, in per se known manner, one either

a) reacts a compound of the general formula II

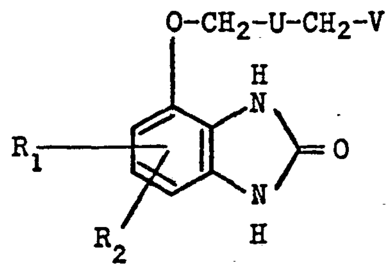

(II),

with a compound of the general formula III

W—R (III)

in which R, $R_1$ and $R_2$ have the above-given meanings and U stands for the group

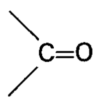

or

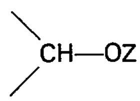

whereby Z possesses the meaning of the substituent $R_3$ or, together with V, can also be a single bond, and one of the residues V and W represents an amino group and the other a reactive residue and, if U signifies the group

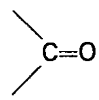

subsequently reduces; or

b) reacts a compound of the general formula IV

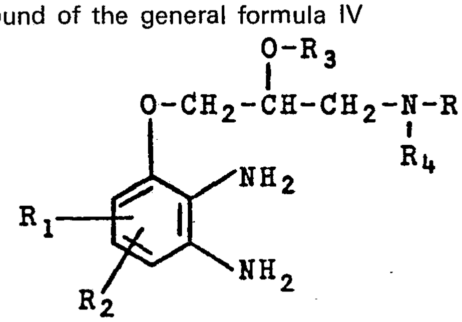

(IV)

with a compound of the general formula V

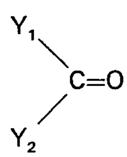

(V)

in which R, $R_1$, $R_2$ and $R_3$ have the above-given meaning while $R_4$ represents a hydrogen atom or a protective group which can be split off, $Y_1$ and $Y_2$ are the same or different and each represents a reactive residue, and subsequently again splits of a protective group $R_4$ possibly present, or

c) reacts a compound of the general formula VI

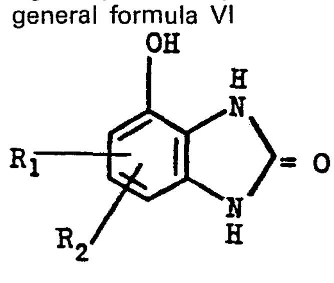

(VI)

with a compound of the general formula:—

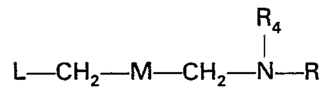

(VII),

in which R, $R_1$, $R_2$ and $R_4$ have the above-given meaning, while M stands for the group

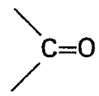 or 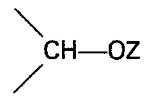

whereby Z possesses the meaning of the substituent $R_3$ or, together with L, can also be a single bond and L represents a reactive residue, if M signifies the group

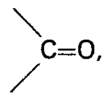

subsequently reduces and again splits off a protective group $R_4$ possibly present, and subsequent to the reactions possible converts compounds of the general formula I into pharmacologically acceptable salts, whereby one possibly previously acylates the hydroxyl group in compounds of the general formula I, in which $R_3$ signifies hydrogen.

3. Medicaments containing a compound according to claim 1, as well as per se known pharmacologically acceptable carrier and adjuvant materials.

4. Compounds according to claim 1 for the treatment or prophylaxis of heart and circulatory diseases.